# EUROPEAN PATENT APPLICATION

(11) **EP 2 042 175 A1**
(43) Date of publication of application: **01.04.2009**
(21) Application number: 07384035.7
(22) Date of filing: 21.09.2007
(51) Int. Cl.: A61K 31/454, A61P 3/04, A61P 3/10

(54) **Dose regimens of CB1-Receptor ligands in the treatment of obesity**

(71) Applicant: Laboratorios del Dr. Esteve S.A., 08041 Barcelona (ES)
(72) Inventor: Buschmann, Helmut H. Dr., 08960 Sant Just Desvem (ES); Vela- Hernandez, José- Miguel, 08028 Barcelona (ES); Fisas- Escasany, Maria Angeles, 08025 Barcelona (ES)
(74) Representative: Peters, Hajo

(57) **Abstract**

The present invention relates to a method of treatment using 5-(4-Chloro-phenyl)-1-(2,4-dichloro-phenyl)-4,5-dihydro-1H-pyrazole-3-carboxylic acid piperidin-1-ylamide as racemate or (R)- and (S)-enantiomer or mixtures thereof in certain dose regimens.

## Description

The present invention relates to a method of treatment using 5-(4-Chloro-phenyl)-(2,4-dichloro-phenyl)-4,5-dihydro-1H-pyrazole-3-carboxylic acid piperidin-1-ylamide as racemate or (R)- and (S)-enantiomer or mixtures thereof in certain dose regimens.

Cannabinoids are compounds, which are derived from the cannabis sativa plant which is commonly known as marijuana. The most active chemical compound of the naturally occurring cannabinoids is tetrahydrocannabinol (THC), particularly Δ⁹-THC.

These naturally occuring cannabinoids as well as their synthetic analogues promote their physiological effects via binding to specific G-coupled receptors, the so-called cannabinoid-receptors.

At present, two distinct types of receptors that bind both the naturally occurring and synthetic cannabinoids have been identified and cloned. These receptors, which are designated CB₁ and CB₂ are involved in a variety of physiological or pathophysiological processes in humans and animals, e.g. processes related to the central nervous system, immune system, cardiovascular system, endocrinous system, respiratory system, the gastrointestinal tract or to reproduction, as described for example, in Hollister, Pharm. Rev. 38, 1986, 1-20; Reny and Singha, Prog. Drug. Res., 36, 71-114, 1991; Consroe and Sandyk, in Marijuana/Cannabinoids, Neurobiology and Neurophysiology, 459, Murphy L. and Barthe A. Eds., CRC Press, 1992.

Therefore, compounds, which have a high binding affinity for these cannabinoid receptors and which are suitable for modulating these receptors are useful in the prevention and/or treatment of cannabinoid-receptor related disorders.

In particular, the CB₁-Receptor is involved in many different food-intake related disorders such as bulimia or obesity, including obesity associated with type II diabetes (non-insulin-dependent diabetes) and thus, compounds suitable for regulating this receptor may be used in the prophylaxis and/or treatment of these disorders.

Also dyslipidaemia, metabolic syndrome (both in its weight dependent or weight independent aspects) and diabetes type II have over the last decades risen in importance for the public health, especially for the developed or developing countries, likely due to a demoscopically marked increase in the proportion of obese or overweight members of the population or in average age.

The compounds according to formula (I), (Ia) or (Ib) (also known as 5-(4-Chloro-phenyl)-1-(2,4-dichloro-phenyl)-4,5-dihydro-1H-pyrazole-3-carboxylic acid piperidin-1-ylamide as racemate and its (S)- and (R)-enantiomers) are described together with ways for their synthesis and their biological activity in applications WO 2005/077911 A1 (racemate), WO 2007/09686 A2 ((S)-enantiomer)and WO 2007/09695 A1 ((R)-enantiomer) respectively; the content of these publications being included here by reference. These compounds were shown to be CB1-binders and/or compounds with antiobesity activity and/or compounds having a reducing effect on triglyceride levels in blood in in-vivo models. Thus, these compounds according to formulas (I), (Ia) and (Ib) did show clear effects especially hinting at a great potential for the treatment or prevention of obesity, dyslipidaemia, metabolic syndrome (both in its weight dependent or weight independent aspects) and/or diabetes type II.

Nevertheless, as a general principle even with these save and effective compounds it still is desirable to reduce the amount of active principle, the pharmaceutically active compound, used in order to either overcome potential side effects or to simply reduce the amount of active principle being consumed, e.g. for cost saving reasons.This is further stressed by the fact that the treatment of e.g. obesity or the reduction of food uptake is often stretched over long periods of time, with the desired effect being normally reached relatively quickly, followed by an extended phase of stabilizing the effect. So, a treatment having more mild effects at the beginning, but finally reaching the same effect, while still using considerable less amount of active compound/principle, might be highly desirable.

Thus, it was an object of the present invention to provide a means for reducing the amount of active principle being used.

Said object was achieved by the use of a pharmaceutical effective amount of an active principle selected from at least one compound of formula (I), (Ia) or (Ib) or mixtures thereof 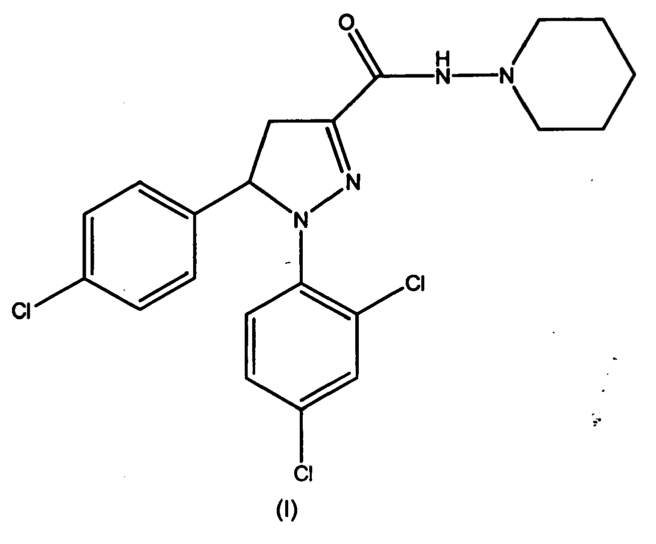 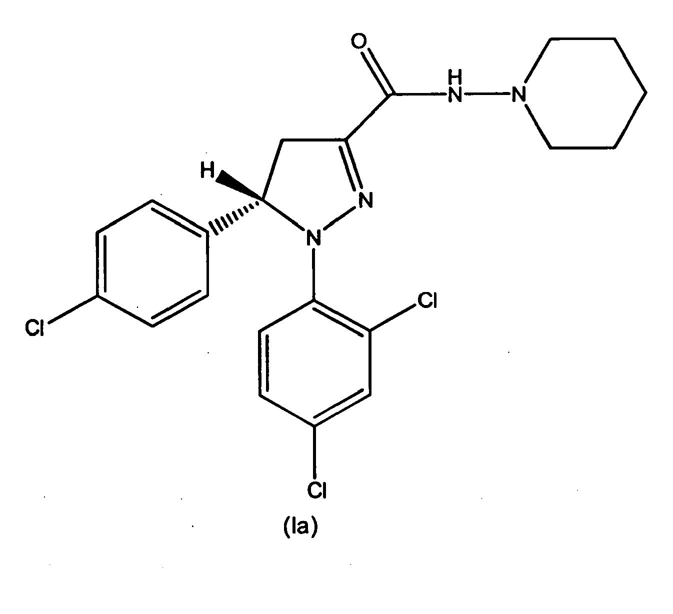 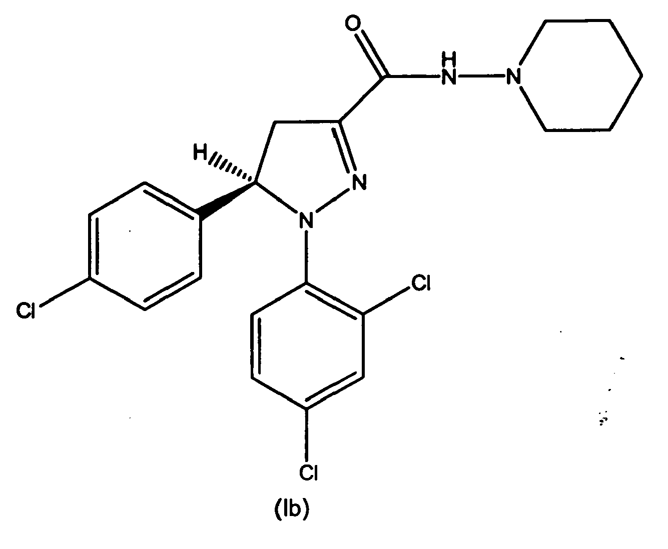 , optionally in form of one or more of the appropriate polymorphs, in amorphous form and/or corresponding salts and/or corresponding solvates thereof,
for the treatment of obesity, appetency disorders, dyslepidemia, drug-induced weight-gain, metabolic syndrome or diabetes type II or for reducing food intake and/or body weight in a mammal in need thereof as a unit dosage form according to a continuous schedule having a dosing interval from 6 times to 1 time per week.

Preferably this use is for the treatment of obesity; or preferably is for the treatment of metabolic syndrome (weight depenent or independent) or dyslepidemia; or preferably is for the treatment of diabetes type II; or preferably is for reducing body weight in a mammal.

It has surprisingly been found that a single comparatively low dose of the compounds of formula (I), (Ia) or (Ib) given less frequently than the usual once or twice daily given dosage form, does result in a long-term effect on body weight. Thus a dosage form of the compound according to formula (I) given only every other day did still result in a significant reduction of body weight in mice compared to vehicle treated animals after. Thus, it might be assumed that following these dose regimens any possible side effect being expectable - especially with long-term treatment - might be avoided, reduced or minimized, while the desired effect is still finally achieved and maintained.

"Unit dosage form" in the sense of this application is defined as any pharmaceutical formulation comprising a certain - optionally varying, but preferably constant - amount of the active principle/s.

"Continuous schedule" in the sense of this application is defined as a therapeutic regimen teaching an ongoing schedule of certain points in time - measured from first treatment - when a unit doage form should be applied to a mammal in need thereof.

"Dosing interval" in the sense of this application is defined as the interval in time between two different applications of the unit dosage form.

The active principle according to the invention may be subject to any particle size reduction like milling, grinding, nanosizing or micronisations, or to complexation like e.g. complexation with Ciclodextrines, or PEG.

Another, parallel aspect of the invention is the use of a pharmaceutical effective amount of an active principle selected from at least one compound of formula (I), (Ia) or (Ib) or mixtures thereof 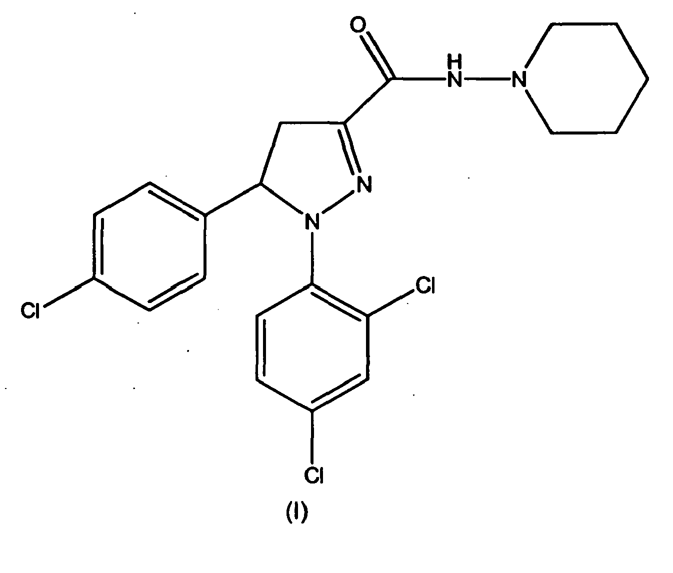 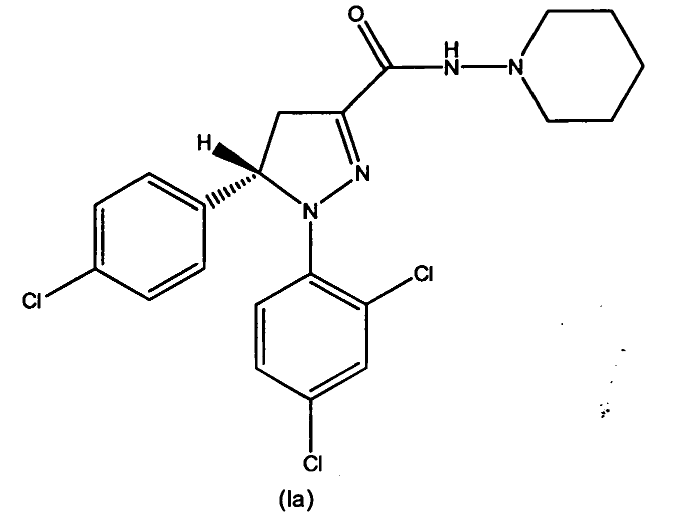 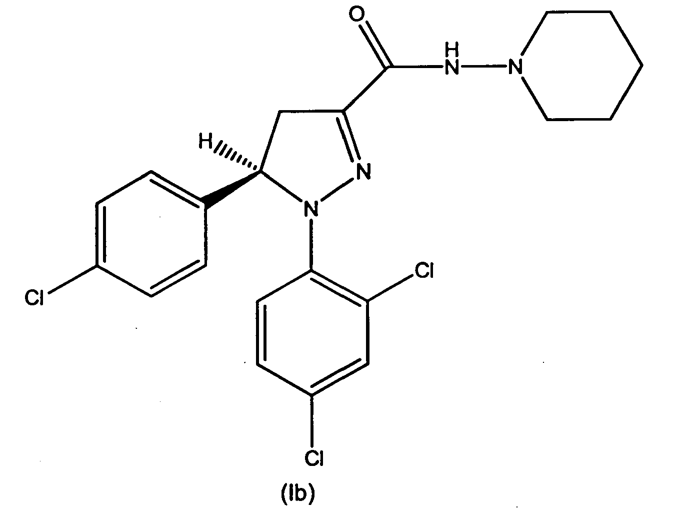 , optionally in form of one or more of the appropriate polymorphs, in amorphous form and/or corresponding salts and/or corresponding solvates thereof,

in the manufacture of a medicament for the treatment of obesity, appetency disorders, dyslepidemia, drug-induced weight-gain, metabolic syndrome or diabetes type II or for reducing food intake and/or body weight in a mammal, wherein said medicament is adapted for administration as a unit dosage form according to a continuous schedule having a dosing interval from 6 times to 1 time per week.

Preferably this use is for the treatment of obesity; or preferably is for the treatment of metabolic syndrome (weight depenent or independent) or dyslepidemia; or preferably is for the treatment of diabetes type II; or preferably is for reducing body weight in a mammal.

A very preferred embodiment of the invention relates to the use according to the invention wherein the dosing interval is selected from the group consisting of once-every-two-days dosing, thrice-weekly dosing, once-every three-days dosing, twice-weekly dosing, once-every-four-days dosing, once-every-five-days dosing, once-every-six-days dosing, or once-weekly dosing; preferably once-every-two-days dosing, thrice-weekly dosing, once-every three-days dosing, twice-weekly dosing, or once-every-four-days dosing.

Another, parallel aspect of the invention is the use of a pharmaceutical effective amount of an active principle selected from at least one compound of formula (I), (Ia) or (Ib) or mixtures thereof 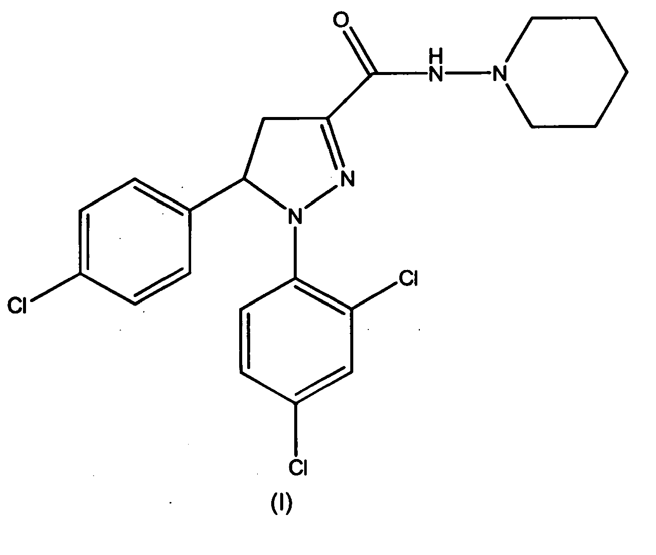 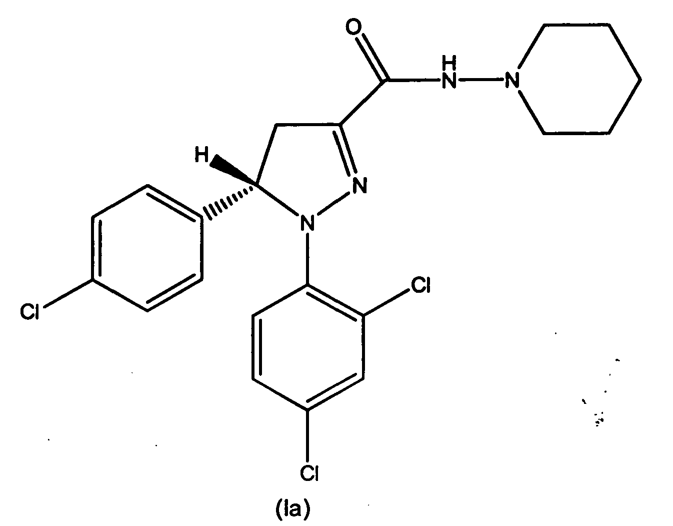 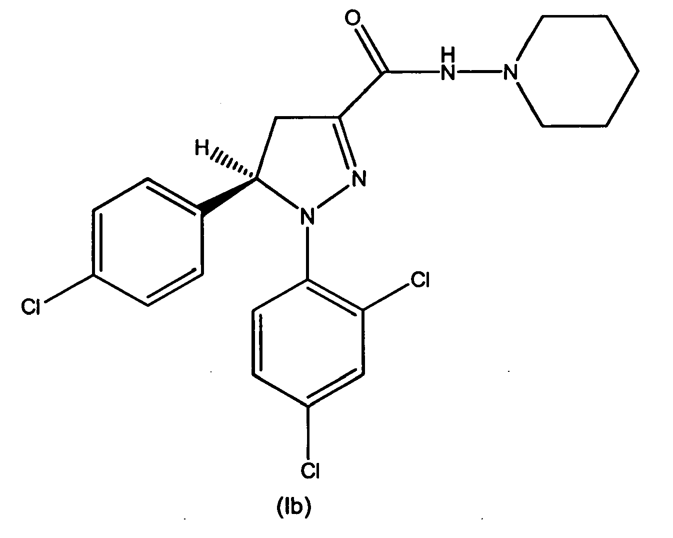 , optionally in form of one or more of the appropriate polymorphs, in amorphous form and/or corresponding salts and/or corresponding solvates thereof,
for the treatment of obesity, appetency disorders, dyslepidemia, drug-induced weight-gain, metabolic syndrome or diabetes type II or for reducing food intake and/or body weight in a mammal as a unit dosage form according to a continuous schedule having a periodicity from about 6 times every week to about once every week. Preferably that said continuous schedule is having a periodicity of once every 2, 3 or 4 days or once every 5, 6 or 7 days, preferably of once every 2, 3 or 4 days.

"Periodicity" in the sense of this application is defined as a repeating interval between two different applications of the unit dosage form.

Preferably this use is for the treatment of obesity; or preferably is for the treatment of metabolic syndrome (weight depenent or independent) or dyslepidemia; or preferably is for the treatment of diabetes type II; or preferably is for reducing body weight in a mammal.

Another very preferred embodiment of the invention is a use according to the invention wherein the unit dosage form is comprising the active principle selected from at least one of
- (rac)-5-(4-Chloro-phenyl)-1-(2,4-dichloro-phenyl)-4,5-dihydro-1H-pyrazole-3-carboxylic acid piperidin-1-ylamide;
- (R)- 5-(4-Chloro-phenyl)-1-(2,4-dichloro-phenyl)-4,5-dihydro-1 H-pyrazole-3-carboxylic acid piperidin-1-ylamide, or
- (S)- 5-(4-Chlro-phenyl)-1-(2,4-dichloro-phenyl)-4,5-dihydro-1H-pyrazole-3-carboxylic acid piperidin-1-ylamide,
- or mixtures thereof;
   optionally in form of one or more of the appropriate polymorphs, in amorphous form and/or corresponding salts and/or corresponding solvates thereof,
in an amount of 1 to 250 mg, preferably 10 to 200 mg, more preferably 15 to 150 mg.

Another very preferred embodiment of the invention is a use according to the invention wherein the unit dosage form is comprising the active principle in an amount of 1 to 250 mg, preferably 10 to 200 mg, more preferably 15 to 150 mg.

Another very preferred embodiment of the invention is a use according to the invention wherein the unit dosage form is comprising the active principle selected from at least one of
- (rac)-5-(4-Chloro-phenyl)-1-(2,4-dichloro-phenyl)-4,5-dihydro-1H-pyrazole-3-carboxylic acid piperidin-1-ylamide;
- (R)- 5-(4-Chloro-phenyl)-1-(2,4-dichloro-phenyl)-4,5-dihydro-1H-pyrazole-3-carboxylic acid piperidin-1-ylamide, or
- (S)- 5-(4-Chloro-phenyl)-1-(2,4-dichloro-phenyl)-4,5-dihydro-1H-pyrazole-3-carboxylic acid piperidin-1-ylamide,
- or mixtures thereof;
   optionally in form of one or more of the appropriate polymorphs, in amorphous form and/or corresponding salts and/or corresponding solvates thereof.

Another very preferred embodiment of the invention is a use according to the invention wherein the unit dosage form is suitable for administering 0.02 to 5 mg/kg, preferably 0.2 to 4 mg/kg, or more preferably 0.3 to 3 mg/kg most preferably 0.25 to 2.5 mg/kg of the active principle to said mammal in need thereof.

Another very preferred embodiment of the invention is a use according to the invention wherein said mammal is a companion animal, such as a cat or dog, or is a human; preferably in that said mammal is a human.

Another very preferred embodiment of the invention is a use according to the invention wherein the unit dosage is in form of a pharmaceutical composition selected from
- a pharmaceutical composition for oral application,
- a pharmaceutical composition for nasal application,
- a pharmaceutical composition for buccal application,
- pharmaceutical composition for rectal application, or
- a pharmaceutical composition for vaginal application;
   or
- a pharmaceutical composition for transdermal application;
- a pharmaceutical composition for systemic application;
   preferably selected from
- a pharmaceutical composition for oral application.

Especially the pharmaceutical composition is selected from
- a tablet,
- a capsule,
- a sachet,
- a powder,
- a caplet,
- a gel,
- a film,
- a pellet,
- a granule,
- an implant,
- a multiparticulate with granules compressed into a tablet,
- a multiparticulate with granules filled into a capsule,
- a multiparticulate with pelets compressed into a tablet,
- a multiparticulate with pelets filled into a capsule, or
- a suppository,
   or
- an injectable solution/dispersion
- a transdermal system like a patch;
preferably selected from
- a tablet,
- a capsule,
- a pellet,
- a granule,
- a multiparticulate with granules compressed into a tablet,
- a multiparticulate with granules filled into a capsule,
- a multiparticulate with pelets compressed into a tablet, or
- a multiparticulate with pelets filled into a capsule.

The pharmaceutical composition may in addition to the active principle also comprise conventional injectable liquid carriers, such as water or suitable alcohols, conventional pharmaceutical excipients for injection, such as stabilising agents, solubilizing agents, and buffers; binding agents, fillers, lubricants, and acceptable wetting agents, or certain additives such as sweeteners, flavoring, preservatives, and emulsifying agents or oils.

In a preferred embodiment of any of the uses according to the invention described above the active principle is selected from
a) (rac)-5-(4-Chloro-phenyl)-1-(2,4-dichloro-phenyl)-4,5-dihydro-1H-pyrazole-3-carboxylic acid piperidin-1-ylamide;
   preferably in
   - form of the α-polymorph;
   - form of the β-polymorph;
   - amorphous form;
   - form of a solvate, preferably a hydrate, more preferably a monohydrate;
   - the free base; or
   - a salt with an acid with a pkₐ ≤ 3.0 especially the acid being selected from 2,5-dihydroxybenzenesulfonic acid, 2-naphthalenesulfonic acid, aspartic acid, benzenesulfonic acid, amphor-10-sulfonic acid, cyclohexylsulfamic acid, dodecylsulfuric acid, ethane-1,2-disulfonic acid, ethanesulfonic acid, fumaric acid, glutamic acid, hydrobromic acid, hydrochloric acid, methanesulfonic acid, naphthalene-1,5-disulfonic acid, nitric acid, phosophoric acid, *p*-toluenesulfonic acid, sulfuric acid and/or thiocyanic acid; more preferably the salt being a hydrochloride;
b) (R)- 5-(4-Chloro-phenyl)-1-(2,4-dichloro-phenyl)-4,5-dihydro-1H-pyrazole-3-carboxylic acid piperidin-1-ylamide, preferably
   preferably in
   - form of the α-polymorph;
   - form of the γ-polymorph;
   - amorphous form;
   - form of a solvate, preferably a hydrate,
   - the free base;
   - a salt with an acid with a pkₐ≤ 3.0,especially the acid being selected from 2,5-dihydroxybenzenesulfonic acid, 2-naphthalenesulfonic acid, aspartic acid, benzenesulfonic acid, camphor-10-suffonic acid, cyclohexylsulfamic acid, dodecylsulfuric acid, ethane-1,2-disulfonic acid, methanesulfonic acid, fumaric acid, glutamic acid, hydrobromic acid, hydrochloric acid, methanesulfonic acid, naphthalene-1,5-disulfonic acid, nitric acid, phosophoric acid, *p*-toluenesulfonic acid, sulfuric acid and/or thiocyanic acid; more preferably the salt being a hydrochloride;
c) (S)- 5-(4-Chloro-phenyl)-1-(2,4-dichloro-phenyl)-4,5-dihydro-1H-pyrazole-3-carboxylic acid piperidin-1-ylamide,
   preferably in
   - form of a polymorph;
   - amorphous form;
   - form of a solvate, preferably a hydrate,
   - the free base;
   - a salt with an acid with a pkₐ≤ 3.0,especially the acid being selected from 2,5-dihydroxybenzenesulfonic acid, 2-naphthalenesulfonic acid, aspartic acid, benzenesulfonic acid, amphor-10-sulfonic acid, cyclohexylsulfamic acid, dodecylsulfuric acid, ethane-1,2-disulfonic acid, ethanesulfonic acid, fumaric acid, glutamic acid, hydrobromic acid, hydrochloric acid, methanesulfonic acid, naphthalene-1,5-disulfonic acid, nitric acid, phosophoric acid, p-toluenesulfonic acid, sulfuric acid and/or thiocyanic acid; more preferably the salt being a hydrochloride;
   or
d) a nonracemic mixtures of (b) and (c).

The γ-polymorph form of (rac)-5-(4-Chloro-phenyl)-1-(2,4-dichloro-phenyl)-4,5-dihydro-1 H-pyrazole-3-carboxylic acid piperidin-1-ylamide including methods for its production is described and exemplified in WO07/17126 the content of which is herwewith enclosed in its entirety by reference.The amoprphous form of (rac)-5-(4-Chloro-phenyl)-1-(2,4-dichloro-phenyl)-4,5-dihydro-1H-pyrazole-3-carboxylic acid piperidin-1-ylamide including methods for its production is described and exemplified in WO07/17124 the content of which is herwewith enclosed in its entirety by reference. A hydrate, especially the mono-hydrate of (rac)-5-(4-Chloro-phenyl)-1-(2,4-dichloro-phenyl)-4,5-dihydro-1 H-pyrazole-3-carboxylic acid piperidin-1-ylamide including methods for its production is described and exemplified in WO07/09705, the content of which is herwewith enclosed in its entirety by reference.

Salts with an acid with a pkₐ ≤ 3.0 of (rac)-5-(4-Chloro-phenyl)-1-(2,4-dichloro-phenyl)-4,5-dihydro-1H-pyrazole-3-carboxylic acid piperidin-1-ylamide, (R)-5-(4-Chloro-phenyl)-1-(2,4-dichloro-phenyl)-4,5-dihydro-1H-pyrazole-3-carboxylic acid piperidin-1-ylamide and (S)-5-(4-Chloro-phenyl)-1-(2,4-dichloro-phenyl)-4,5-dihydro-1H-pyrazole-3-carboxylic acid piperidin-1-ylamide are described and exemplified in WO07/09708 the content of which is herwewith enclosed in its entirety by reference. Specifically mentioned are salts of the the acid being selected from 2,5-dihydroxybenzenesulfonic acid, 2-naphthalenesulfonic acid, aspartic acid, benzenesulfonic acid, amphor-10-sulfonic acid, cyclohexylsulfamic acid, dodecylsulfuric acid, ethane-1,2-disulfonic acid, ethanesulfonic acid, fumaric acid, glutamic acid, hydrobromic acid, hydrochloric acid, methanesulfonic acid, naphthalene-1,5-disulfonic acid, nitric acid, phosophoric acid, *p*-toluenesulfonic acid, sulfuric acid and/or thiocyanic acid.
Some salts like e.g. hydrochloride salts etc. of (rac)-5-(4-Chloro-phenyl)-1-(2,4-dichloro-phenyl)-4,5-dihydro-1H-pyrazole-3-carboxylic acid piperidin-1-ylamide, (R)-5-(4-Chloro-phenyl)-1-(2,4-dichloro-phenyl)-4,5-dihydro-1H-pyrazole-3-carboxylic acid piperidin-1-ylamide and (S)-5-(4-Chloro-phenyl)-1-(2,4-dichloro-phenyl)-4,5-dihydro-1H-pyrazole-3-carboxylic acid piperidin-1-ylamide the have also been described in their respective applications WO 2005/077911 A1, WO 2007/09695 A1 and WO 2007/09686 A2.

The α-polymorph of (rac)-5-(4-Chloro-phenyl)-1-(2,4-dichloro-phenyl)-4,5-dihydro-1 H-pyrazole-3-carboxylic acid piperidin-1-ylamide is defined by its physico-chemical attributes from the following list:
- it crystallizes as a triclinic cell with an α-angle of 78° and/or a β-angle of 74° and a γ-angle of 88°;
- it melts
   above 180°C, preferably at an onset point of 183 ± 1°C, more preferably at an onset point of 183-184 °C
   and/or
   with a melting peak at 185 ± 2°C, preferably at 184 ± 1°C;
- it decomposes at or above 280 °C;
- it shows in the FTRaman spectrum bands, preferably intense bands, at 2932 ± 5 cm⁻¹ and/or 1647 ± 5 cm⁻¹ and/or 1593 ± 5 cm⁻¹ and/or 1394 ± 5 cm⁻¹ and/or 1269 ± 5 cm⁻¹ and/or 1253 ± 5 cm⁻¹ and/or 1109 ± 5 cm⁻¹ and/or 1084 ± 5 cm⁻¹ and/or 1047 ± 5 cm⁻¹ and/or 783 ± 5 cm⁻¹ and/or 661 ± 5 cm⁻¹ and/or a Raman shift at 1253 ± 5 cm⁻¹ and/or at 1084 ± 5 cm⁻¹ and/or at 533 ± 5 cm⁻¹ and/or and at 364 cm⁻¹

In the process for the manufacture of the α-Polymorph of (rac)-N-piperidinyl-5-(4-chlorophenyl)-1-(2,4-dichlorophenyl)-4,5-dihydro-1H-pyrazole-3-carboxamide a solution of (rac)-N-piperidinyl-5-(4-chlorophenyl)-1-(2,4-dichlorophenyl)-4,5-dihydro-1H-pyrazole-3-carboxamide in an alcohol; preferably ethanol or methanol; is evaporated at room temperature (25 °C) to yield the α-Polymorph of (rac)-N-piperidinyl-5-(4-chlorophenyl)-1-(2,4-dichlorophenyl)-4,5-dihydro-1H-pyrazole-3-carboxamide.

The α-Polymorph of (R)-N-piperidinyl-5-(4-chlorophenyl)-1-(2,4-dichlorophenyl)-4,5-dihydro-1H-pyrazole-3-carboxamide is defined by its physico-chemical attributes from the following list:
- it crystallizes as a monoclinic cell with a beta angle of 104.52° at 100 K;
- it melts
   above 114°C, preferably at an onset point of 118 ± 4°C, more preferably at an onset point of 118±3°C
   and/or
   with a melting peak at 124 ± 6°C, preferably at 124 ± 5 °C,
   and/or
   with a fusion enthalpy of -58 ± 4. J/g, preferably -58 ± 3.0 J/g;
- it decomposes at or above 280 °C;
- it shows in the FTIR spectrum peaks, preferably intense peaks, at 3324 ± 5 cm⁻¹ and/or 1534 ± 5 cm⁻¹;
- it shows in the FTRaman spectrum bands, preferably intense bands, at 3326 ± 5 cm⁻¹ and/or a Raman shift at 3326 ± 5 cm⁻¹.

The process for the manufacture of the α-Polymorph of (R)-N-piperidinyl-5-(4-chlorophenyl)-1-(2,4-dichlorophenyl)-4,5-dihydro-1H-pyrazole-3-carboxamide involves the following steps:
a) in a solution step (R)-N-piperidinyl-5-(4-chlorophenyl)-1-(2,4dichlorophenyl)-4,5-dihydro-1H-pyrazole-3-carboxamide is dissolved in diisopropyl ether, heated to 68°C until (R)-N-piperidinyl-5-(4-chlorophenyl)-1-(2,4-dichlorophenyl)-4,5-dihydro-1H-pyrazole-3-carboxamide is complete dissolved, and
b) in a cooling step done in two phases the solution first is subsequently cooled to 20 ± 2 °C, and then to 0 ± 2 °C, to yield the α-Polymorph of (R)-N-piperidinyl-5-(4-chlorophenyl)-1-(2,4-dichlorophenyl)-4,5-dihydro-1H-pyrazole-3-carboxamide.

The amorphous phase of -(R)-N-piperidinyl-5-(4-chlorophenyl)-1-(2,4-dichlorophenyl)-4,5 dihydro-1H-pyrazole-3-carboxamide is defined by its physico-chemical attributes from the following list:
- it shows a glass transition in the DSC analysis at 64.5 ± 4 °C, preferably at 64.5 ± 3 °C, more preferably at 64.5 ± 2 °C or also between 63 and 66° C;
- it decomposes at or above 300 °C;
- it shows in the FTIR spectrum peaks, preferably intense and/or broad peaks at 1656 ± 5 cm⁻¹ and/or 1473 ± 5 cm⁻¹ and no peaks, preferably no intense peaks, at 3325 ±10 cm⁻¹;
- it shows in the FTRaman spectrum bands, preferably intense and/or broad bands, at 1666±5cm⁻¹

In the process for the manufacture of the amorphous phase of (R)-N-piperidinyl-5-(4-chlorophenyl)-1-(2,4-dichlorophenyl)-4,5-dihydro-1H-pyrazole-3-carboxamide (R)-N-piperidinyl-5-(4-chlorophenyl)-1-(2,4dichlorophenyl)-4,5-dihydro-1H-pyrazole-3-carboxamide is melted, preferably at a temperature between 78 and 88°C, and subsequently cooled to below the melting point, preferably to room temperature.

The γ-Polymorph of (R)-N-piperidinyl-5-(4-chlorophenyl)-1-(2,4-dichlorophenyl)-4,5-dihydro-1 H-pyrazole-3-carboxamide is defined by its physico-chemical attributes from the following list:
- it crystallizes as a monoclinic cell with a beta angle of 92.15° at room temperature and/or a beta angle of 92.41° at 100 K;
- it melts
   above 120°C, preferably at an onset point of 122 ± 2 °C, more preferably at an onset point of 122 ±1 °C,
   and/or
   with a melting peak at 127 ± 3 °C, preferably at 127 ± 2 °C, more preferably at 127 ± 1.5 °C,
   and/or
   with a fusion enthalpy of -62.5 ± 3.0 J/g, preferably -62.5 ± 2.0 J/g, or preferably - 62.5 ± 1.3 J/g;
- it decomposes at or above 299 °C;
- it shows in the FTIR spectrum peaks, preferably intense peaks, at 1665 ± 5 cm⁻¹ and no peaks, preferably no intense peaks, at 3325 ±10 cm⁻¹;
- it shows in the FTRaman spectrum bands, preferably intense bands, at 1666 ± 5 cm⁻¹ and/or 1585 ± 5 cm⁻¹ and/or a Raman shift at 1666 ± 5 cm⁻¹.

The process for the manufacture of the γ-Polymorph of (R)-N-piperidinyl-5-(4-chlorophenyl)-1-(2,4-dichlorophenyl)-4,5-dihydro-1H-pyrazole-3-carboxamide involves the following steps:
a) in a solution step (R)-N-piperidinyl-5-(4-chlorophenyl)-1-(2,4dichlorophenyl)-4,5-dihydro-1H-pyrazole-3-carboxamide is dissolved in diisopropyl ether, heated to 68°C until (R)-N-piperidinyl-5-(4-chlorophenyl)-1-(2,4-dichlorophenyl)-4,5-dihydro-1H-pyrazole-3-carboxamide is complete dissolved, and
b) in a cooling step done in two phases the solution first is subsequently cooled to 20 ± 2 °C, and then to 0 ± 2 °C, to yield the γ-Polymorph of (R)-N-piperidinyl-5-(4-chlorophenyl)-1-(2 ,4-dichlorophenyl)-4,5-dihydro-1H-pyrazole-3-carboxamide.

Another aspect of the invention provides a method for the treatment of obesity, appetency disorders, dyslepidemia, drug-induced weight-gain, metabolic syndrome or diabetes type II or for reducing food intake and/or body weight in a mammal in need thereof comprising administering to said mammal a pharmaceutical effective amount of an active principle selected from at least one compound of formula (I), (Ia) or (Ib) or mixtures thereof 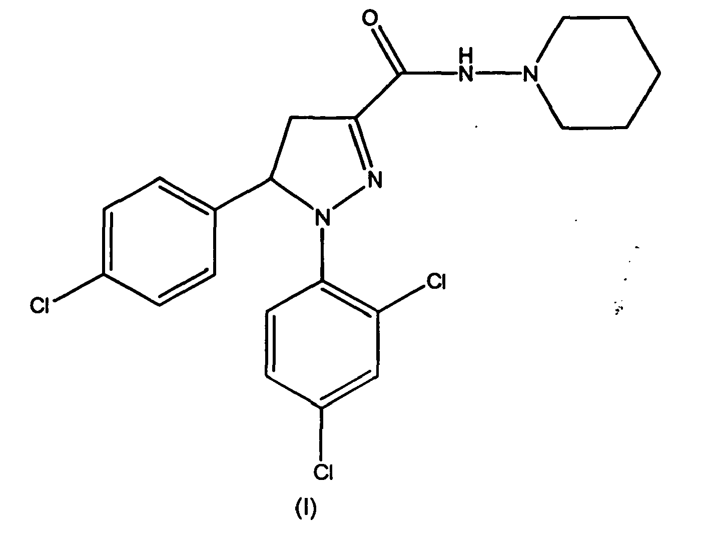 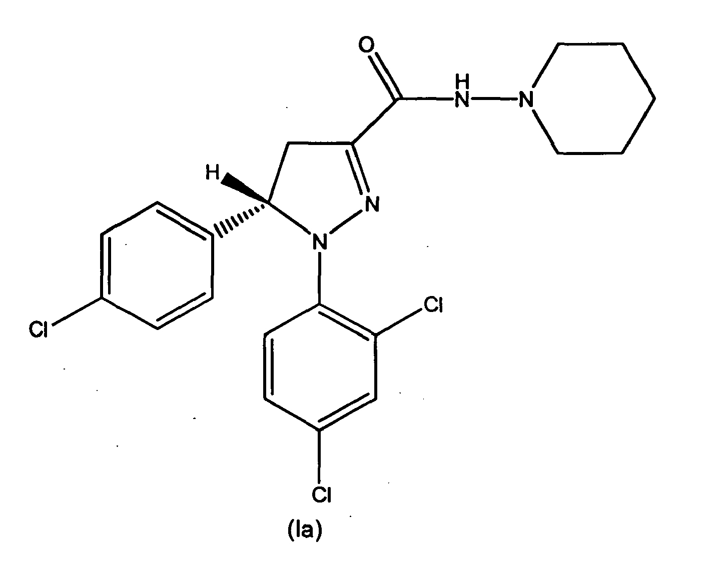 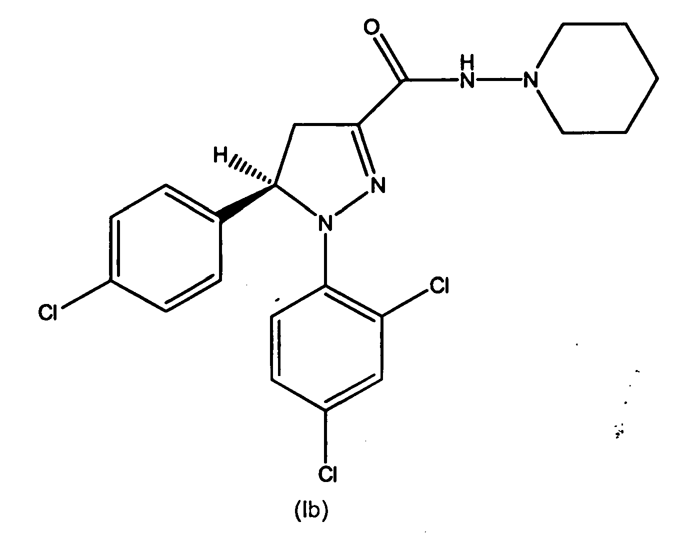 , optionally in form of one or more of the appropriate polymorphs, in amorphous form and/or corresponding salts and/or corresponding solvates thereof,
as a unit dosage form according to a continuous schedule having a dosing interval from 6 times to 1 time per week. Preferably the dosing interval is selected from the group consisting of once-every-two-days dosing, thrice-weekly dosing, once-every three-days dosing, twice-weekly dosing, once-every-four-days dosing, once-every-five-days dosing, once-every-six-days dosing, or once-weekly dosing; more preferably once-every-two-days dosing, thrice-weekly dosing, once-every three-days dosing, or twice-weekly dosing.

Preferably this method is for the treatment of obesity; or preferably is for the treatment of metabolic syndrome (weight depenent or independent) or dyslepidemia; or preferably is for the treatment of diabetes type II; or preferably is for reducing body weight in a mammal.

Another parallel aspect of the invention provides a method for the treatment of obesity, appetency disorders, dyslepidemia, drug-induced weight-gain, metabolic syndrome or diabetes type II or for reducing food intake and/or body weight in a mammal in need thereof comprising administering to said mammal a pharmaceutical effective amount of an active principle selected from at least one compound of formula (I), (Ia) or (Ib) or mixtures thereof 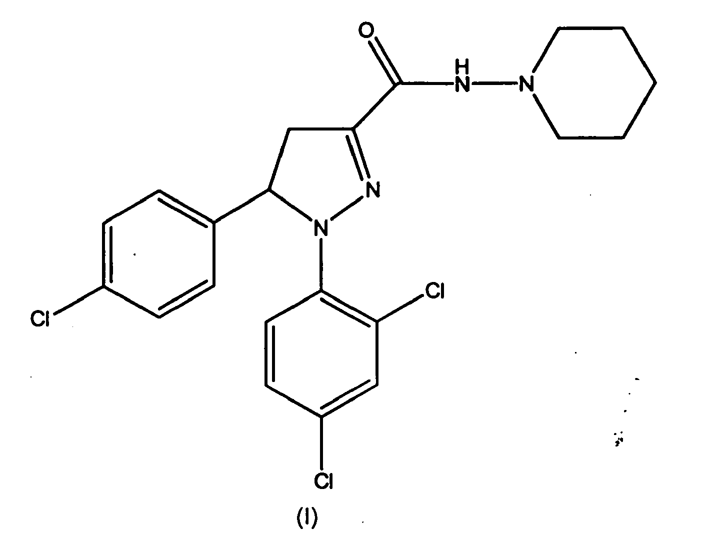 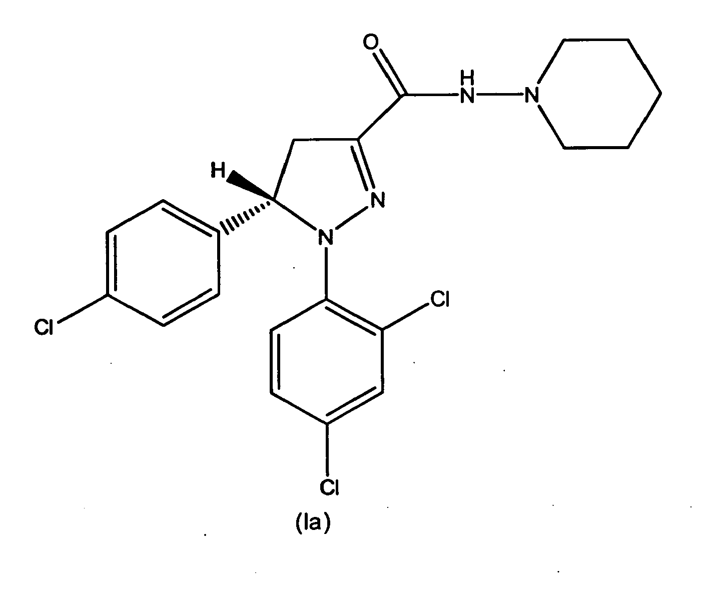 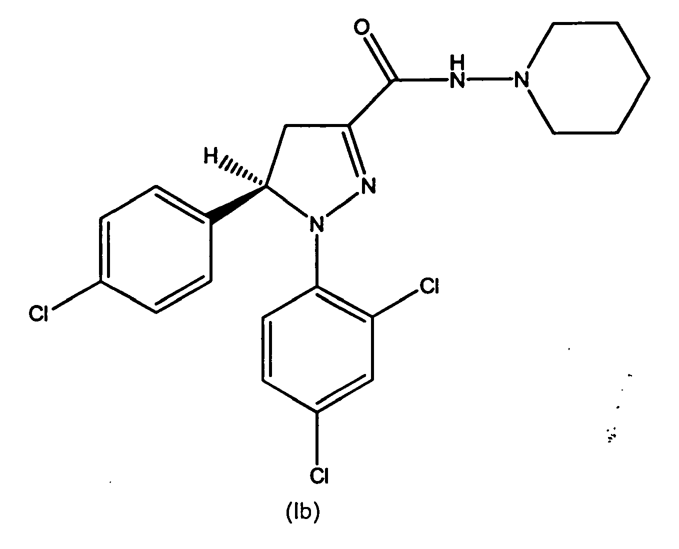 optionally in form of one or more of the appropriate polymorphs, in amorphous form and/or corresponding salts and/or corresponding solvates thereof,
once every 2 to 7 days. Preferably said continuous schedule is having a periodicity of once every 2, 3 or 4 days or once every 5, 6 or 7 days, more preferably of once every 2, 3 or 4 days.

Preferably this method is for the treatment of obesity; or preferably is for the treatment of metabolic syndrome (weight depenent or independent) or dyslepidemia; or preferably is for the treatment of diabetes type II; or preferably is for reducing body weight in a mammal.

In a preferred embodiment of the method according to the invention the pharmaceutical effective amount of the active principle selected from at least one of
- (rac)-5-(4-Chloro-phenyl)-1-(2,4-dichloro-phenyl)-4,5-dihydro-1H-pyrazole-3-carboxylic acid piperidin-1-ylamide;
- (R)- 5-(4-Chloro-phenyl)-1-(2,4-dichloro-phenyl)-4,5-dihydro-1 H-pyrazole-3-carboxylic acid piperidin-1-ylamide, or
- (S)- 5-(4-Chloro-phenyl)-1-(2,4-dichloro-phenyl)-4,5.dihydro-1H-pyrazole-3-carboxylic acid piperidin-1-ylamide,
- or mixtures thereof;
   optionally in form of one or more of the appropriate polymorphs, in amorphous form and/or corresponding salts and/or corresponding solvates thereof,
administered is an amount of 1 to 250 mg, preferably 10 to 200 mg, more preferably 15 to 150 mg.

In a preferred embodiment of the method according to the invention the pharmaceutical effective amount of the active principle administered is an amount of 1 to 250 mg, preferably 10 to 200 mg, more preferably 15 to 150 mg.

In a preferred embodiment of the method according to the invention the pharmaceutical effective amount of the active principle selected from at least one of
- (rac)-5-(4-Chloro-phenyl)-1-(2,4-dichloro-phenyl)-4,5-dihydro-1H-pyrazole-3-carboxylic acid piperidin-1-ylamide;
- (R)- 5-(4-Chloro-phenyl)-(2,4-dichloro-phenyl)-4,5-dihydro-1H-pyrazole-3-carboxylic acid piperidin-1-ylamide, or
- (S)- 5-(4-Chloro-phenyl)-1-(2,4-dichloro-phenyl)-4,5-dihydro-1H-pyrazole-3-carboxylic acid piperidin-1-ylamide,
- or mixtures thereof;
   optionally in form of one or more of the appropriate polymorphs, in amorphous form and/or corresponding salts and/or corresponding solvates thereof.

In a preferred embodiment of the method according to the invention the pharmaceutical effective amount of the active principle administered is 0.02 to 5 mg/kg, preferably 0.2 to 4 mg/kg, or more preferably 0.3 to 3 mg/kg most preferably 0.25 to 2.5 mg/kg of the active principle.

In a preferred embodiment of the method according to the invention said mammal is a companion animal, such as a cat or dog, or is a human; preferably in that said mammal is a human.

In a preferred embodiment of the method according to the invention the pharmaceutical effective amount of the active principle is in form of a pharmaceutical composition selected from
- a pharmaceutical composition for oral application,
- a pharmaceutical composition for nasal application,
- a pharmaceutical composition for buccal application,
- a pharmaceutical composition for rectal application, or
- a pharmaceutical composition for vaginal application;
   or
- a pharmaceutical composition for transdermal application;
- a pharmaceutical composition for systemic application;
preferably selected from
- a pharmaceutical composition for oral application.

Preferably the pharmaceutical composition is selected from
- a tablet,
- a capsule,
- a sachet,
- a powder,
- a caplet,
- a gel,
- a film,
- a pellet,
- a granule,
- an implant,
- a multiparticulate with granules compressed into a tablet,
- a multiparticulate with granules filled into a capsule,
- a multiparticulate with pelets compressed into a tablet,
- a multiparticulate with pelets filled into a capsule, or
- a suppository,
   or
- an injectable solution/dispersion
- a transdermal system like a patch;
preferably selected from
- a tablet,
- a capsule,
- a pellet,
- a granule,
- a multiparticulate with granules compressed into a tablet,
- a multiparticulate with granules filled into a capsule,
- a multiparticulate with pelets compressed into a tablet, or
- a multiparticulate with pelets filled into a capsule.

In a preferred embodiment of the method according to the invention the active principle is selected from
a) (rac)-5-(4-Chloro-phenyl)-1-(2 ,4-dichloro-phenyl)-4,5-dihydro-1H-pyrazole-3-carboxylic acid piperidin-1-ylamide;
   preferably in
   - form of the α-polymorph;
   - form of the β-polymorph;
   - amorphous form;
   - form of a solvate, preferably a hydrate, more preferably a monohydrate;
   - the free base; or
   - a salt with an acid with a pkₐ≤ 3.0,especially the acid being selected from 2,5-dihydroxybenzenesulfonic acid, 2-naphthalenesulfonic acid, aspartic acid, benzenesulfonic acid, amphor-10-sulfonic acid, cyclohexylsulfamic acid, dodecylsulfuric acid, ethane-1,2-disuffonic acid, ethanesulfonic acid, fumaric acid, glutamic acid, hydrobromic acid, hydrochloric acid, methanesulfonic acid, naphthalene-1,5-disulfonic acid, nitric acid, phosophoric acid, p-toluenesulfonic acid, sulfuric acid and/or thiocyanic acid; more preferably the salt being a hydrochloride;
b) (R)- 5-(4-Chloro-phenyl)-1-(2,4-dichloro-phenyl)-4,5-dihydro-1H-pyrazole-3-carboxylic acid piperidin-1-ylamide, preferably
   preferably in
   - form of the α-polymorph;
   - form of the γ-polymorph;
   - amorphous form;
   - form of a solvate, preferably a hydrate,
   - the free base;
   - a salt with an acid with a pkₐ ≤ 3.0,especially the acid being selected from 2,5-dihydroxybenzenesulfonic acid, 2-naphthalenesulfonic acid, aspartic acid, benzenesulfonic acid, amphor-10-sulfonic acid, cyclohexylsulfamic acid, dodecylsulfuric acid, ethane-1,2-disulfonic acid, ethanesulfonic acid, fumaric acid, glutamic acid, hydrobromic acid, hydrochloric acid, methanesulfonic acid, naphthalene-1,5-disulfonic acid, nitric acid, phosophoric acid, p-toluenesulfonic acid, sulfuric acid and/or thiocyanic acid; more preferably the salt being a hydrochloride;
c) (S)- 5-(4-Chloro-phenyl)-1-(2,4-dichloro-phenyl)-4,5-dihydro-1H-pyrazole-3-carboxylic acid piperidin-1-ylamide,
   preferably in
   - form of a polymorph;
   - amorphous form;
   - form of a solvate, preferably a hydrate,
   - the free base;
   - a salt with an acid with a pkₐ ≤ 3.0,especially the acid being selected from 2,5-dihydroxybenzenesulfonic acid, 2-naphthalenesulfonic acid, aspartic acid, benzenesulfonic acid, amphor-10-sulfonic acid, cyclohexylsulfamic acid, dodecylsulfuric acid, ethane-1,2-disulfonic acid, ethanesulfonic acid, fumaric acid, glutamic acid, hydrobromic acid, hydrochloric acid, methanesulfonic acid, naphthalene-1,5-disulfonic acid, nitric acid, phosophoric acid, *p*-toluenesulfonic acid, sulfuric acid and/or thiocyanic acid; more preferably the salt being a hydrochloride;
   or
d) a nonracemic mixtures of (b) and (c).

The following part of the description is exemplifying the invention and is not meant to limit it.

### Examples:

### IN-VIVO TESTS:

### Effects of (rac)-5-(4-Chloro-phenyl)-1-(2,4-dichloro-phenyl)-4,5-dihydro-1H-pyrazole-3-carboxylic acid piperidin-1-ylamide on body weight

The in-vivo testing for antiobesic activity of the inventive pyrazoline compounds is carried out orientated on the method described in the publication of G. Colombo et al., "Appetite Suppression and Weight Loss after the Cannabinoid Antagonist SR 141716"; Life Sciences, 63 (8), 113-117, (1998). The respective part of the description is hereby incorporated by reference and forms part of the present disclosure.

### Animals

The experiment was performed on male Wistar rats (originally in the weight range 250-300 g). The rats were housed in pairs in polypropylene cages with solid floors and sawdust bedding at a temperature of 21±4°C and 55±20% humidity. Animals were maintained on a reverse phase light-dark cycle (lights off for 8 h) during which time the room was illuminated by red light. Animals were fed a high-fat-diet over the time of the whole experiment. Animals were housed in pairs.

### Experimental procedures for the feeding study

On the start of the study, animals were weighed (to the nearest 0.1 g using an electronic top-pan balance) and allocated into weight-matched treatment groups, each containing 10 animals. Following this, animals were either treated once a day with vehicle (0.5% hydroxy-propyl-methylcellulose [HPMC]; 5 ml/kg) p.o. or every second day on day 0, 2, 4, 6 and 8 with the test drug, (rac)-5-(4-Chloro-phenyl)-1-(2,4-dichloro-phenyl)-4,5-dihydro-1 H-pyrazole-3-carboxylic acid piperidin-1-ylamide (30 mg/kg p.o.) in 5 ml/kg of vehicle.

Body weight was measured once daily. Variations in body weight were accounted for.

Statistical comparisons between the body weights of the different treatment groups have been made by analysis of covariance followed by Dunnett's test to compare the treatment group with the vehicle-treated controls. For the body weight data (body weights each day), the weights of each animals on Day 0 (the day of the first drug or vehicle treatment) was determined and considered as 100 % and the body weight measured each day and expressed as percentage of change over the basic weight on day 0. Thus, the means were adjusted for differences in body weight between the groups on Day 0 and standard errors of the mean (SEM) were calculated from the residuals of the statistical model. On each day following treatment the difference in the mean of the differences in body weight between the vehicle- and the drug-treated-group was significant.

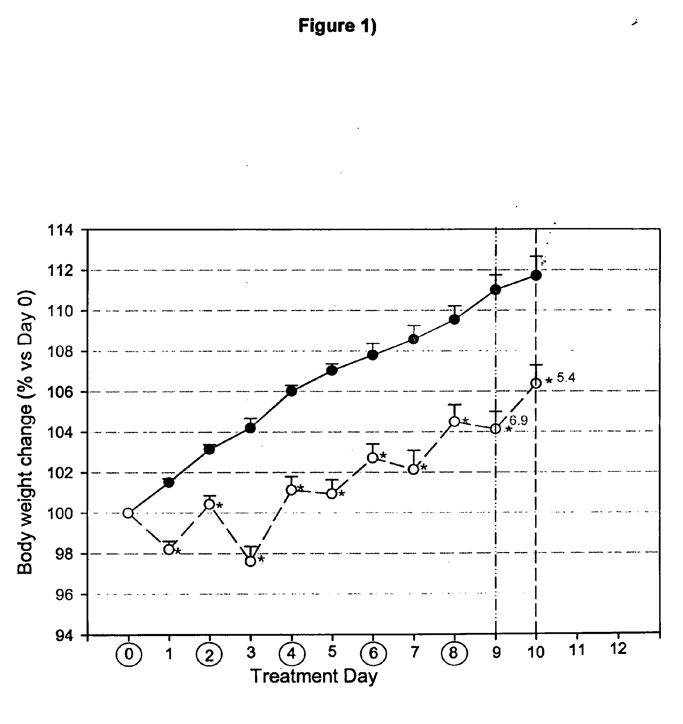

The results can be seen in Figure 1) with the differences in body weight achieved with the test compound (lower curve) over vehicle (upper curve) after 9 or 10 days respectively being significant with 6.9% (on day 9) or 5.4% (on day 10) respectively. The days of treatment with the test compound (rac)-5-(4-Chloro-phenyl)-1-(2,4-dichloro-phenyl)-4,5-dihydro-1H-pyrazole-3-carboxylic acid piperidin-1-ylamide (30 mg/kg p.o.) on the x-axis are circled.

### COMPOUND EXAMPLES:

### Compound Example 1:

### α-Polymorph of (rac)-N-piperidinyl-5-(4-chlorophenyl)-1-(2,4-dichlorophenyl)-4,5-dihydro-1 H-pyrazole-3-carboxamide

### C1(a):

The α-Polymorph was obtained by evaporation of a solution of N-piperidinyl-5-(4-chlorophenyl)-1-(2,4-dichlorophenyl)-4,5-dihydro-1H-pyrazole-3-carboxamide in ethanol at room temperature (25°C).

### C1(b):

The α-Polymorph was obtained by evaporation of a solution of N-piperidinyl-5-(4-chlorophenyl)-1-(2,4-dichlorophenyl)-4,5-dihydro-1H-pyrazole-3-carboxamide in methanol at room temperature (25°C).

### C1(c):

### Crystal structure of the α-Polymorph of N-piperidinyl-5-(4-chlorophenyl)-1-(2,4-dichlorophenyl)-4,5-dihydro-1H-pyrazole-3-carboxamide

Crystals suitable for X-ray single crystal determination could be obtained from methanol and ethanol (also dimethylformamide) by slow evaporation at room temperature. Typically the α-polymorph of N-piperidinyl-5-(4-chlorophenyl)-1-(2,4-dichlorophenyl)-4,5-dihydro-1H-pyrazole-3-carboxamide grows in form of twinned needles.

A selected crystal was tested using an X-ray single crystal structure determination.

### Procedure:

The measured crystals were selected using a Zeiss stereomicroscope using polarized light and prepared under inert conditions immersed in perfloropolyether as protecting oil for manipulation. Crystal structure determination was carried out using a Bruker-Nonius diffractometer equipped with a APPEX 2 4K CCD area detector, a FR591 rotating anode with Mo_{kα} radiation, Montel mirrors as monochromator and a Kyroflex low temperature. device (T = 100K). Fullsphere data collection omega and phi scans. Analysis was done with software.

The α-Polymorph of N-piperidinyl-5-(4-chlorophenyl)-1-(2,4-dichlorophenyl)-4,5-dihydro-1H-pyrazole-3-carboxamide crystallizes in a triclinic cell.

A summary of the cell constants is given in table 1 below:

| | Unit cell dimensions | |
|---|---|---|
| Triclinic *P* 1 | a = 9.6749(11) Å | α = 78.112(3)° |
| Volume: 2121.3(4) Å³ | b = 13.1273(17) Å | β = 74.449(3)° |
| Density: 1.415 Mg/m³ | C = 17.725 (2) Å | γ = 88.447(3)° |

### Compound Example 2:

### α-Polymorph of (R)-N-piperidinyl-5-(4-chlorophenyl)-1-(2,4-dichlorophenyl)-4,5-dihydro-1 H-pyrazole-3-carboxamide

### C2(a):

A solution of (R)-N-piperidinyl-5-(4-chlorophenyl)-1-(2,4-dichlorophenyl)-4,5-dihydro-1H-pyrazole-3-carboxamide in 300 mL of diisopropyl ether was stirred at 450 rpm and heated to a gentle reflux (68°C) under nitrogen athmosphere. To the resulting mixture, diisopropyl ether was added dropwise (2 hours) to dissolve the solid, and the resulting mixture solution was cooled at 20°C in 90 minutes. A white solid precipitated on cooling. The suspension was left 3 hours at 20°C under a gentle stirring (200 rpm), cooled to 0°C in 2 hours and left overnight at that temperature. The crystals formed were filtered off to give white solid wich correspond to the crystalline phase of the α-Polymorph of (R)-N-piperidinyl-5-(4-chlorophenyl)-1-(2,4-dichlorophenyl)-4,5-dihydro-1H-pyrazole-3-carboxamide.

### C2(b):

### Crystal structure of the α-Polymorph of (R)-N-piperidinyl-5-(4-chlorophenyl)-1-(2,4-dichlorophenyl)-4,5-dihydro-1H-pyrazole-3-carboxamide

The crystals of the α-Polymorph are growing in form of needles which are partially twinned. On a crystal an X-ray single crystal structure determination was done. The α-polymorph crystallizes in a monoclinic cell. A summary of the cell constants is given in table 1:

**Table 1: Cell constants of α-polymorph at 100 K**

| | Unit cell dimensions | |
|---|---|---|
| Monoclinic *P*2₁ | a = 13.6460(13) A | α = 90°. |
| Volume: 2158.8(4) Å³ | b = 11.6896(12) A | β = 104.519(2)°. |
| Density: 1.390 Mg/m³ | c = 13.9800(14) A | γ = 90° |

### Compound Example 3:

### Amorphous Phase of (R)-N-piperidinyl-5-(4-chlorophenyl)-1-(2,4-dichlorophenyl)-4,5-dihydro-1H-pyrazole-3-carboxamide

### C3(a):

The amorphous phase is obtained by the following way:

A sample of (R)-N-piperidinyl-5-(4-chlorophenyl)-1-(2,4-dichlorophenyl)-4,5-dihydro-1H-pyrazole-3-carboxamide is heated to 80°C (a temperature above the melting point of 75-78 °C see above) until it is completely melted. Subsequently it is cooled to room temperature until it is completely solid again to yield the amorphous phase of (R)-N-piperidinyl-5-(4-chlorophenyl)-1-(2,4-dichlorophenyl)-4,5-dihydro-1H-pyrazole-3-carboxamide.

### C3(b):

### DSC analysis of the amorphous phase of (R)-N-piperidinyl-5-(4-chlorophenyl)-1-(2,4-dichlorophenyl)-4,5-dihydro-1H-pyrazole-3-carboxamide

The DSC analysis of the *amorphous phase* presents a base line step between 60 and 70°C corresponding to the glass transition (Tg). No other peak is observed at higher temperatures, indicating that, at least during the analysis, this phase does not crystallize. Since the Tg is a reversible phenomenon, it can be better measured if first the sample is heated to a temperature above the Tg, is then cooled at the same rate to room temperature, and is again heated above the Tg. The true onset of the Tg for the amorphous phase is at 63-66°C.

### Compound Example 4:

### γ-Polymorph of (R)-N-piperidinyl-5-(4-chlorophenyl)-1-(2,4-dichlorophenyl)-4,5-dihydro-1H-pyrazole-3-carboxamide

### C4(a):

A solution of (R)-N-piperidinyl-5-(4-chlorophenyl)-1-(2,4-dichlorophenyl)-4,5-dihydro-1H-pyrazole-3-carboxamide in 300 mL of diisopropyl ether was stirred at 450 rpm and heated to a gentle reflux (68°C) under nitrogen athmosphere. To the resulting mixture, 185 mL diisopropyl ether was added dropwise (2 hours) to dissolve the solid, and the resulting mixture solution was cooled at 20°C in 90 minutes. A white solid precipitated on cooling. The suspension was left 3 hours at 20°C under a gentle stirring (200 rpm), cooled to 0°C in 2 hours and left overnight at that temperature. The crystals formed were filtered off to give white solid (14.99 g, 83% yield) wich correspond to the crystalline phase of the γ-Polymorph of (R)-N-piperidinyl-5-(4-chlorophenyl)-1-(2,4-dichlorophenyl)-4,5-dihydro-1H-pyrazole-3-carboxamide.

### C4(b):

### Crystal structure of the γ-Polymorph of (R)-N-piperidinyl-5-(4-chlorophenyl)-1-(2,4-dichlorophenyl)-4,5-dihydro-1H-pyrazole-3-carboxamide

The crystals of the γ-Polymorph are growing in form of solid blocks. After selecting a crystal fragment an X-ray single crystal structure determination was done. The measurement of the crystal was done at room temperature. The γ-polymorph crystallizes in a monoclinic cell with a β-angle of 92.15°. The orthorhombic disposition, which would mean a β-angle of exactly 90°. gives a non acceptable statistic. A summary of the cell constants is given in table 1:

**Table 1: Cell constants of γ-polymorph at room temperature**

| | Unit cell dimensions | |
|---|---|---|
| Monoclinic *P*2₁ | a = 9.7447(5) Å | α = 90°. |
| Volume: 2200.80(19) Å³ | b = 18.873S(9) Å | β = 92.1500(10)°. |
| Density: 1.363 Mg/m³ | c = 11.9747(6) Å | γ = 90° |

Additionally, the cell dimensions of the γ-polymorph were determined at 100 K (see table 2).

**Table 2: Cell constants of γ-polymorph at 100 K**

| | **Unit cell dimensions** | |
|---|---|---|
| Monoclinic *P*2₁ | a = 9.719(4) Å | α = 90°. |
| Volume: 2158(3) A³ | b = 18.683(7) Å | β = 92.41 (2)°. |
| Density: 1.390 Mg/m³ | c = 11.896(4) Å | γ = 90° |

## Claims

1. Use of a pharmaceutical effective amount of an active principle selected from at least one compound of formula (I), (Ia) or (Ib) or mixtures thereof 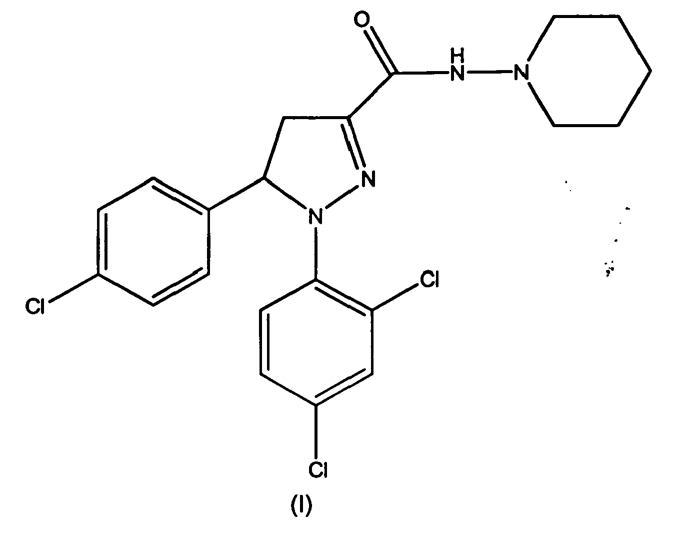 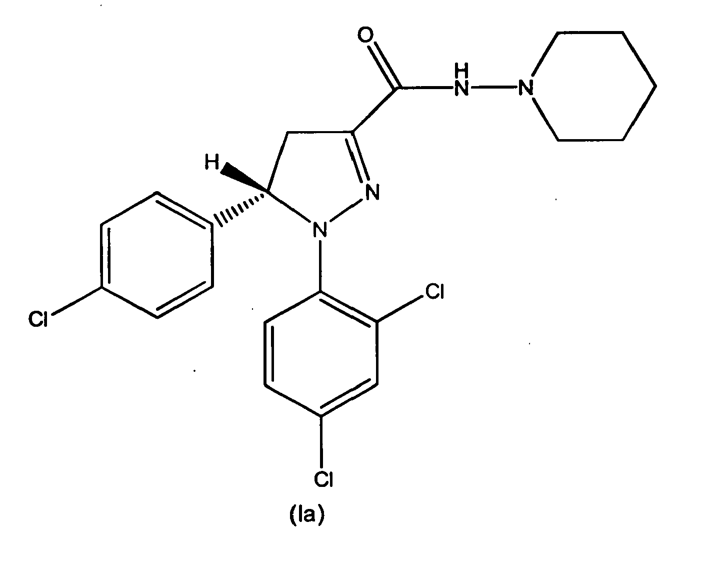 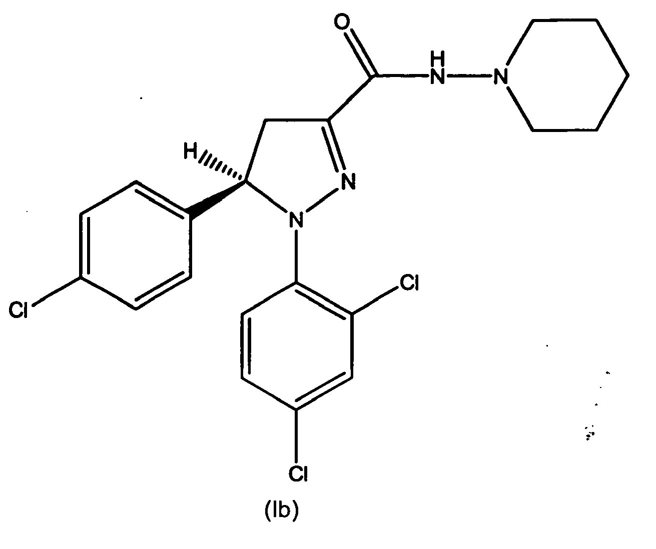 , optionally in form of one or more of the appropriate polymorphs, in amorphous form and/or corresponding salts and/or corresponding solvates thereof,
for the treatment of obesity, appetency disorders, dyslepidemia, drug-induced weight-gain, metabolic syndrome or diabetes type II or for reducing food intake and/or body weight in a mammal in need thereof as a unit dosage form according to a continuous schedule having a dosing interval from 6 times to 1 time per week.

2. Use of a pharmaceutical effective amount of an active principle selected from at least one compound of formula (I), (Ia) or (Ib) or mixtures thereof 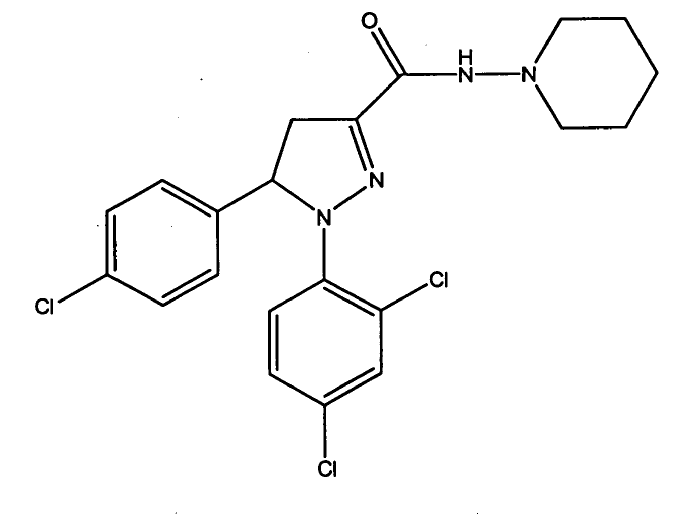 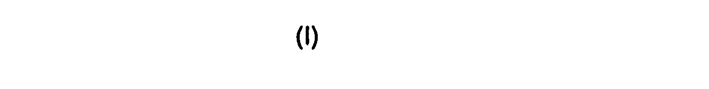 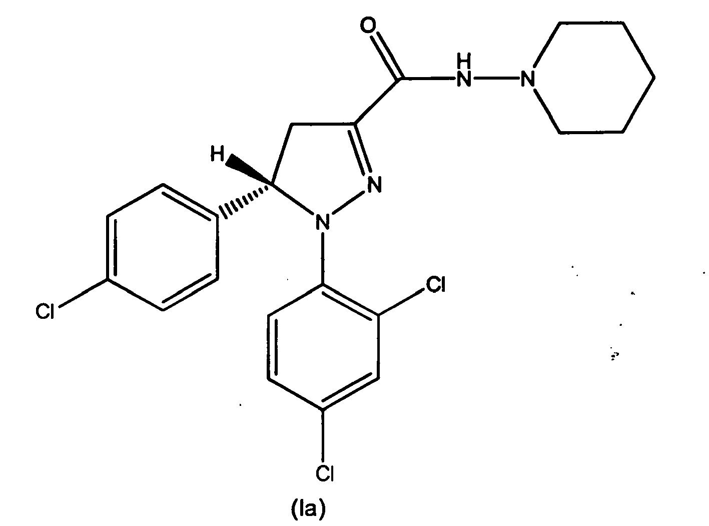 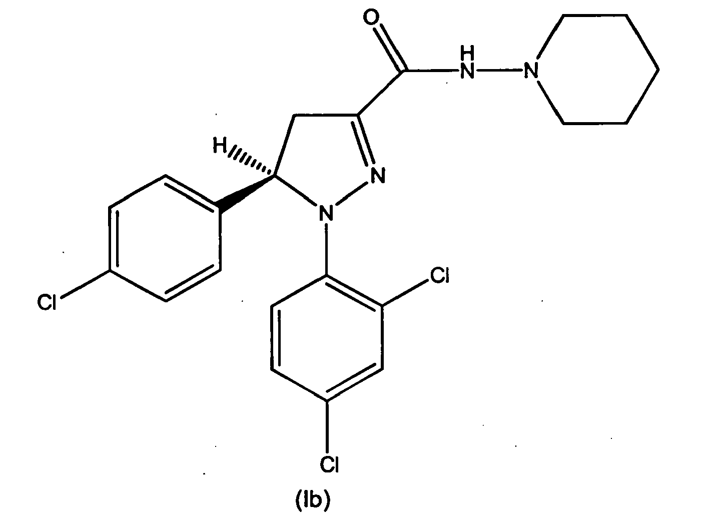 , optionally in form of one or more of the appropriate polymorphs, in amorphous form and/or corresponding salts and/or corresponding solvates thereof,
in the manufacture of a medicament for the treatment of obesity, appetency disorders, dyslepidemia, drug-induced weight-gain, metabolic syndrome or diabetes type II or for reducing food intake and/or body weight in a mammal, wherein said medicament is adapted for administration as a unit dosage form according to a continuous schedule having a dosing interval from 6 times to 1 time per week.

3. Use according to any of claims 1 or 2, **characterized in that** the dosing interval is selected from the group consisting of once-every-two-days dosing, thrice-weekly dosing, once-every three-days dosing, twice-weekly dosing, once-every-four-days dosing, once-every-five-days dosing, once-every-six-days dosing, or once-weekly dosing; preferably once-every-two-days dosing, thrice-weekly dosing, once-every three-days dosing, twice-weekly dosing, or once-every-four-days dosing.

4. Use of a pharmaceutical effective amount of an active principle selected from at least one compound of formula (I), (Ia) or (Ib) or mixtures thereof 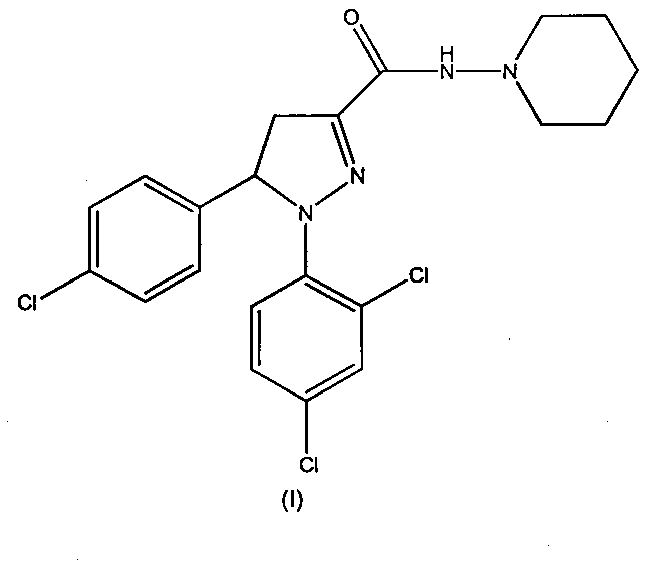 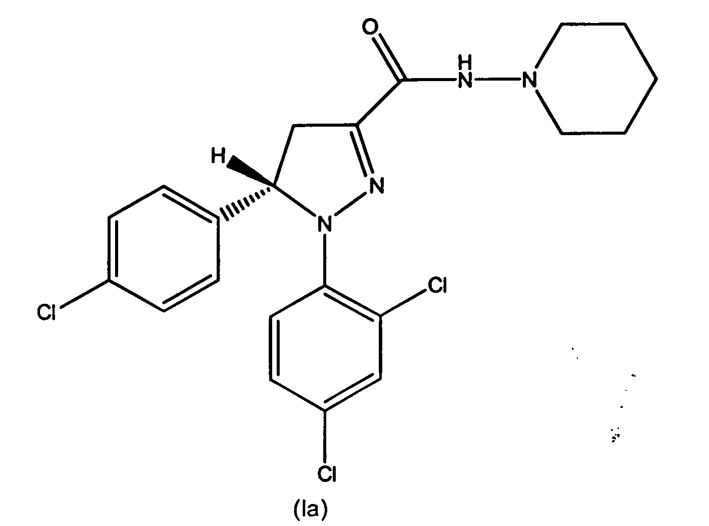 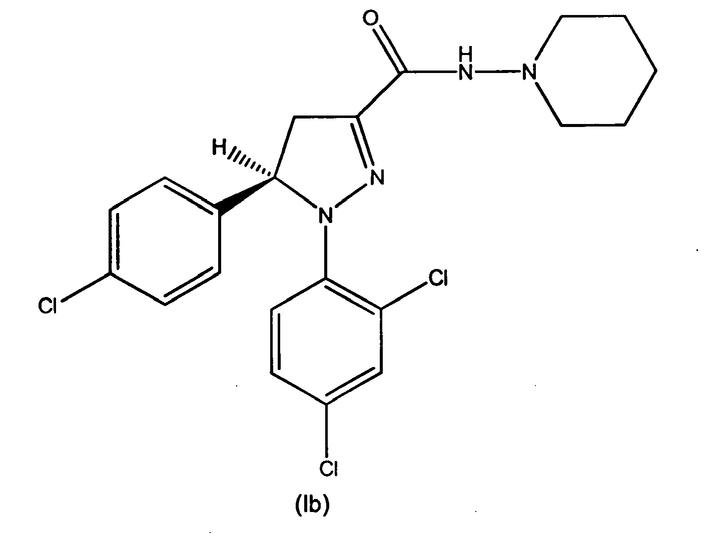 optionally in form of one or more of the appropriate polymorphs, in amorphous form and/or corresponding salts and/or corresponding solvates thereof,
for the treatment of obesity, appetency disorders, dyslepidemia, drug-induced weight-gain, metabolic syndrome or diabetes type II or for reducing food intake and/or body weight in a mammal as a unit dosage form according to a continuous schedule having a periodicity from about 6 times every week to about once every week.

5. Use according to claim 3 **characterized in that** said continuous schedule is having a periodicity of once every 2, 3 or 4 days or once every 5, 6 or 7 days, preferably of once every 2, 3 or 4 days.

6. Use according to any of claims 1 to 5, **characterized in that** the unit dosage form is comprising the active principle selected from at least one of
• rac)-5-(4-Chloro-phenyl)-1-(2,4-dichloro-phenyl)-4,5-dihydro-1H-pyrazole-3-carboxylic acid piperidin-1-ylamide;
• (R)- 5-(4-Chloro-phenyl)-1-(2,4-dichloro-phenyl)-4,5-dihydro-1H-pyrazole-3-carboxylic acid piperidin-1-ylamide, or
• (S)- 5-(4-Chloro-phenyl)-1-(2,4-dichloro-phenyl)-4,5-dihydro-1 H-pyrazole-3-carboxylic acid piperidin-1-ylamide,
• or mixtures thereof;
optionally in form of one or more of the appropriate polymorphs, in amorphous form and/or corresponding salts and/or corresponding solvates thereof,
in an amount of 1 to 250 mg, preferably 10 to 200 mg, more preferably 15 to 150 mg.

7. Use according to any of claims 1 to 6, **characterized in that** the unit dosage form is suitable for administering 0.02 to 5 mg/kg, preferably 0.2 to 4 mg/kg, or more preferably 0.3 to 3 mg/kg most preferably 0.25 to 2.5 mg/kg of the active principle to said mammal in need thereof.

8. Use according to any of claims 1 to 7, **characterized in that** said mammal is a companion animal, such as a cat or dog, or is a human; preferably **in that** said mammal is a human.

9. Use according to any of claims 1 to 8, **characterized in that** the unit dosage is in form of a pharmaceutical composition selected from
• a pharmaceutical composition for oral application,
• a pharmaceutical composition for nasal application,
• a pharmaceutical composition for buccal application,
• a pharmaceutical composition for rectal application, or
• a pharmaceutical composition for vaginal application;
or
• a pharmaceutical composition for transdermal application;
• a pharmaceutical composition for systemic application;
• preferably selected from
• a pharmaceutical composition for oral application.

10. Use according to claim 9, **characterized in that** the pharmaceutical composition is selected from
• tablet,
• a capsule,
• a sachet,
• a powder,
• a caplet,
• a gel,
• a film,
• a pellet,
• a granule,
• an implant,
• a multiparticulate with granules compressed into a tablet,
• a multiparticulate with granules filled into a capsule,
• a multiparticulate with pelets compressed into a tablet,
• a multiparticulate with pelets filled into a capsule, or
• a suppository,
or
• an injectable solution/dispersion
• a transdermal system like a patch;
preferably selected from
• a tablet,
• a capsule,
• a pellet,
• a granule,
• a multiparticulate with granules compressed into a tablet,
• a multiparticulate with granules filled into a capsule,
• a multiparticulate with pelets compressed into a tablet, or
• a multiparticulate with pelets filled into a capsule.

11. Use according to any of claims 1 to 10, **characterized in that** the active principle is selected from
a) (rac)-5-(4-Chloro-phenyl)-1-(2,4-dichloro-phenyl)-4,5-dihydro-1H-pyrazole-3-carboxylic acid piperidin-1-ylamide;
preferably in
• form of the α-polymorph;
• form of the β-polymorph;
• amorphous form;
• form of a solvate, preferably a hydrate, more preferably a monohydrate;
• the free base; or
• a salt with an acid with a pkₐ ≤ 3.0,especially the acid being selected from 2,5-dihydroxybenzenesulfonic acid, 2-naphthalenesulfonic acid, aspartic acid, benzenesulfonic acid, amphor-10-sulfonic acid, cyclohexylsulfamic acid, dodecylsulfuric acid, ethane-1,2-disulfonic acid, ethanesulfonic acid, fumaric acid, glutamic acid, hydrobromic acid, hydrochloric acid, methanesulfonic acid, naphthalene-1,5-disulfonic acid, nitric acid, phosophoric acid, p-toluenesulfonic acid, sulfuric acid and/or thiocyanic acid; more preferably the salt being a hydrochloride;
b) (R)- 5-(4-Chloro-phenyl)-1-(2,4-dichloro-phenyl)-4,5-dihydro-1H-pyrazole-3-carboxylic acid piperidin-1-ylamide, preferably
preferably in
• form of the α-polymorph;
• form of the γ-polymorph;
• amorphous form;
• form of a solvate, preferably a hydrate,
• the free base;
• a salt with an acid with a pkₐ ≤ 3.0,especially the acid being selected from 2,5-dihydroxybenzenesulfonic acid, 2-naphthalenesulfonic acid, aspartic acid, benzenesulfonic acid, amphor-10-sulfonic acid, cyclohexylsulfamic acid, dodecylsulfuric acid, ethane-1,2-disulfonic acid, ethanesulfonic acid, fumaric acid, glutamic acid, hydrobromic acid, hydrochloric acid, methanesulfonic acid, naphthalene-1,5-disulfonic acid, nitric acid, phosophoric acid, *p*-toluenesulfonic acid, sulfuric acid and/or thiocyanic acid; more preferably the salt being a hydrochloride;
c) (S)- 5-(4-Chloro-phenyl)-1-(2,4-dichloro-phenyl)-4,5-dihydro-1H-pyrazole-3-carboxylic acid piperidin-1-ylamide,
preferably in
• form of a polymorph;
• amorphous form;
• form of a solvate, preferably a hydrate,
• the free base;
• a salt with an acid with a pkₐ ≤ 3.0,especially the acid being selected from 2,5-dihydroxybenzenesulfonic acid, 2-naphthalenesulfonic acid, aspartic acid, benzenesulfonic acid, amphor-10-suffonic acid, cyclohexylsulfamic acid, dodecylsulfuric acid, ethane-1,2-disutfonic acid, ethanesulfonic acid, fumaric acid, glutamic acid, hydrobromic acid, hydrochloric acid, methanesulfonic acid, naphthalene-1,5-disulfonic acid, nitric acid, phosophoric acid, p-toluenesulfonic acid, sulfuric acid and/or thiocyanic acid; more preferably the salt being a hydrochloride;
or
d) a nonracemic mixtures of (b) and (c).

12. A method for the treatment of obesity, appetency disorders, dyslepidemia, drug-induced weight-gain, metabolic syndrome or diabetes type II or for reducing food intake and/or body weight in a mammal in need thereof comprising administering to said mammal a pharmaceutical effective amount of an active principle selected from at least one compound of formula (I), (Ia) or (Ib) or mixtures thereof 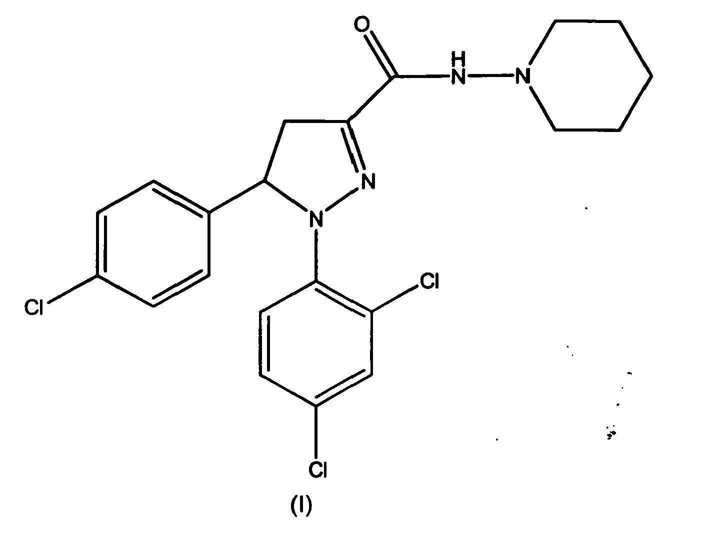 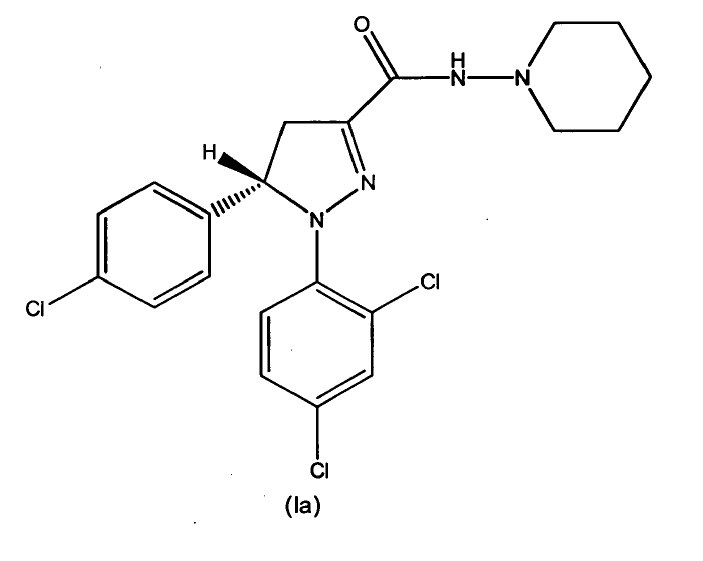 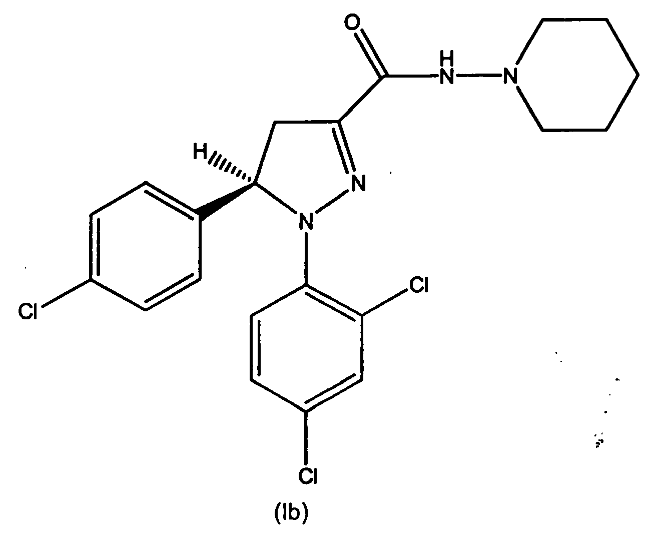 , optionally in form of one or more of the appropriate polymorphs, in amorphous form and/or corresponding salts and/or corresponding solvates thereof,
as a unit dosage form according to a continuous schedule having a dosing interval from 6 times to 1 time per week.

13. Method according to claim 12, **characterized in that** the dosing interval is selected from the group consisting of once-every-two-days dosing, thrice-weekly dosing, once-every three-days dosing, twice-weekly dosing, once-every-four-days dosing, once-every-five-days dosing, once-every-six-days dosing, or once-weekly dosing; preferably once-every-two-days dosing, thrice-weekly dosing, once-every three-days dosing, or twice-weekly dosing.

14. A method for the treatment of obesity, appetency disorders, dyslepidemia, drug-induced weight-gain, metabolic syndrome or diabetes type II or for reducing food intake and/or body weight in a mammal in need thereof comprising administering to said mammal a pharmaceutical effective amount of an active principle selected from at least one compound of formula (I), (Ia) or (Ib) or mixtures thereof 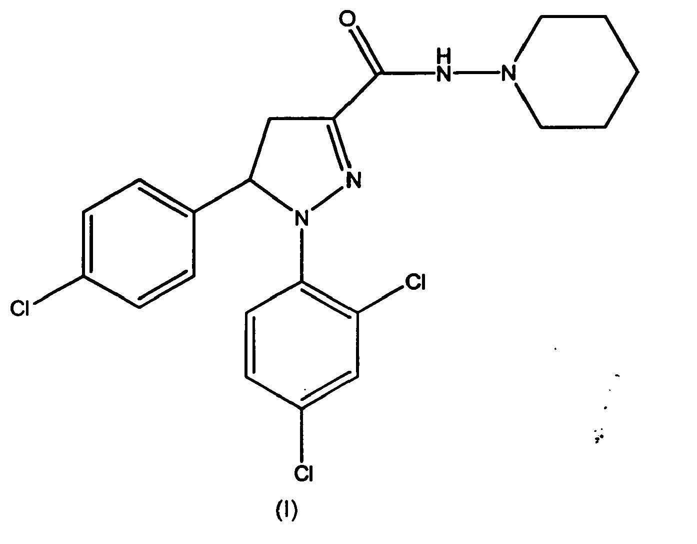 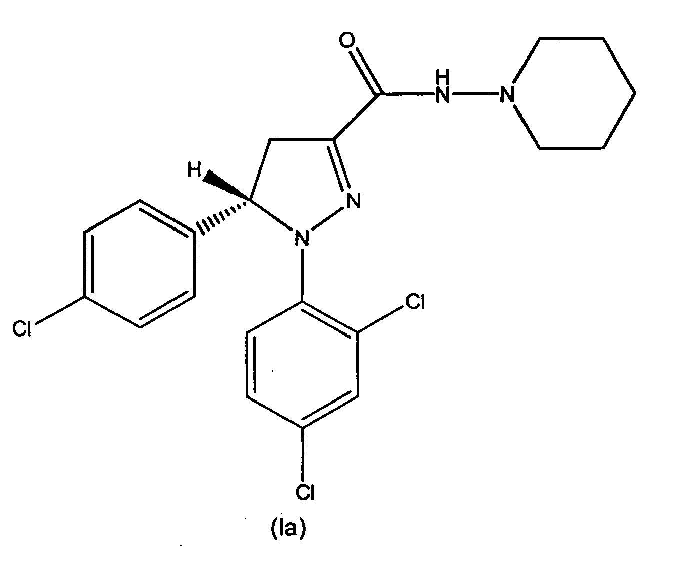 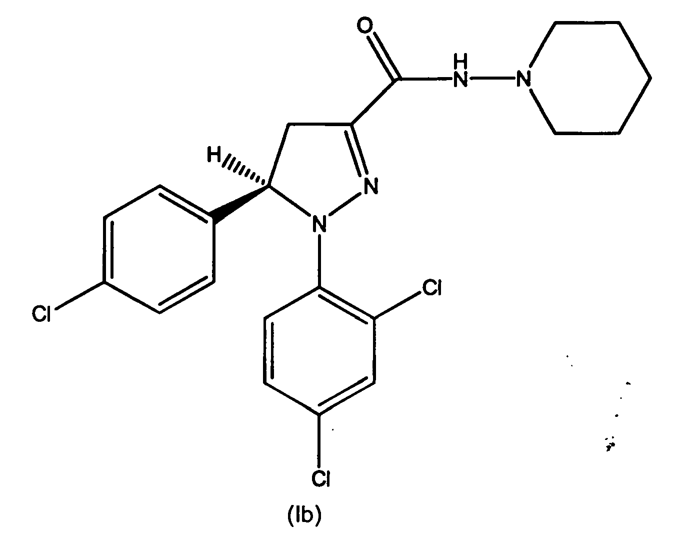 , optionally in form of one or more of the appropriate polymorphs, in amorphous form and/or corresponding salts and/or corresponding solvates thereof,
once every 2 to 7 days.

15. Method according to claim 14 **characterized in that** said continuous schedule is having a periodicity of once every 2, 3 or 4 days or once every 5, 6 or 7 days, preferably of once every 2, 3 or 4 days.

16. Method according to any of claims 12 to 15, **characterized in that** the pharmaceutical effective amount of the active principle selected from at least one of
• (rac)-5-(4-Chloro-pheny)-1-(2,4-dichloro-pheny)-4,5-dihydro-1 H-pyrazole-3-carboxylic acid piperidin-1-ylamide;
• (R)- 5-(4-Chloro-phenyl)-1-(2,4-dichloro-phenyl)-4,5-dihydro-1H-pyrazole-3-carboxylic acid piperidin-1-ylamide, or
• (S)- 5-(4-Chloro-phenyl)-1-(2,4-dichloro-phenyl)-4,5-dihydro-1H-pyrazole-3-carboxylic acid piperidin-1-ylamide,
• or mixtures thereof;
optionally in form of one or more of the appropriate polymorphs, in amorphous form and/or corresponding salts and/or corresponding solvates thereof, administered is an amount of 1 to 250 mg, preferably 10 to 200 mg, more preferably 15 to 150 mg.

17. Method according to any of claims 12 to 16, **characterized in that** the pharmaceutical effective amount of the active principle administered is 0.02 to 5 mg/kg, preferably 0.2 to 4 mg/kg, or more preferably 0.3 to 3 mg/kg most preferably 0.25 to 2.5 mg/kg of the active principle.

18. Method according to any of claims 12 to 17, **characterized in that** said mammal is a companion animal, such as a cat or dog, or is a human; preferably **in that** said mammal is a human.

19. Method according to any of claims 12 to 18, **characterized in that** the pharmaceutical effective amount of the active principle is in form of a pharmaceutical composition selected from
• a pharmaceutical composition for oral application,
• a pharmaceutical composition for nasal application,
• a pharmaceutical composition for buccal application,
• a pharmaceutical composition for rectal application, or
• a pharmaceutical composition for vaginal application;
or
• a pharmaceutical composition for transdermal application;
• a pharmaceutical composition for systemic application;
preferably selected from
• a pharmaceutical composition for oral application.

20. Method according to claim 19, **characterized in that** the pharmaceutical composition is selected from
• a tablet,
• a capsule,
• a sachet,
• a powder,
• a caplet,
• a gel,
• a film,
• a pellet,
• a granule,
• an implant,
• a multiparticulate with granules compressed into a tablet,
• a multiparticulate with granules filled into a capsule,
• a multiparticulate with pelets compressed into a tablet,
• a multiparticulate with pelets filled into a capsule, or
• a suppository,
or
• an injectable solution/dispersion
• a transdermal system like a patch;
preferably selected from
• a tablet,
• a capsule,
• a pellet,
• a granule,
• a multiparticulate with granules compressed into a tablet,
• a multiparticulate with granules filled into a capsule,
• a multiparticulate with pelets compressed into a tablet, or
• a multiparticulate with pelets filled into a capsule.

21. Method according to any of claims 12 to 20, **characterized in that** the active principle is selected from
a) (rac)-5-(4-Chloro-phenyl)-1-(2,4-dichloro-phenyl)-4,5-dihydro-1H-pyrazole-3-carboxylic acid piperidin-1-ylamide;
preferably in
• form of the α-polymorph;
• form of the β-polymorph;
• amorphous form;
• form of a solvate, preferably a hydrate, more preferably a monohydrate;
• the free base; or
• a salt with an acid with a pkₐ ≤ 3.0,especially the acid being selected from 2,5-dihydroxybenzenesulfonic acid, 2-naphthalenesulfonic acid, aspartic acid, benzenesulfonic acid, amphor-10-sufonic acid, cyclohexylsulfamic acid, dodecylsulfuric acid, ethane-1,2-disulfonic acid, ethanesulfonic acid, fumaric acid, glutamic acid, hydrobromic acid, hydrochloric acid, methanesulfonic acid, naphthalene-1,5-disulfonic acid, nitric acid, phosophoric acid, p-toluenesulfonic acid, sulfuric acid and/or thiocyanic acid; more preferably the salt being a hydrochloride;
b) (R)- 5-(4-Chloro-phenyl)-1-(2,4-dichloro-pheny)-4,5-dihydro-1H-pyrazole-3-carboxylic acid piperidin-1-ylamide, preferably
preferably in
• form of the α-polymorph;
• form of the γ-polymorph;
• amorphous form;
• form of a solvate, preferably a hydrate,
• the free base;
• a salt with an acid with a pkₐ ≤ 3.0,especially the acid being selected from 2,5-dihydroxybenzenesulfonic acid, 2-naphthalenesulfonic acid, aspartic acid, benzenesulfonic acid, amphor-10-sulfonic acid, cyclohexylsulfamic acid, dodecylsulfuric acid, ethane-1,2-disulfonic acid, ethanesulfonic acid, fumaric acid, glutamic acid, hydrobromic acid, hydrochloric acid, methanesulfonic acid, naphthalene-1,5-disulfonic acid, nitric acid, phosophoric acid, *p*-toluenesulfonic acid, sulfuric acid and/or thiocyanic acid; more preferably the salt being a hydrochloride;
c) (S)-5-(4-Chloro-phenyl)-1-(2,4-dichloro-phenyl)-4,5-dihydro-1H-pyrazole-3-carboxylic acid piperidin-1-ylamide,
preferably in
• form of a polymorph;
• amorphous form;
• form of a solvate, preferably a hydrate,
• the free base;
• a salt with an acid with a pkₐ ≤ 3.0,especially the acid being selected from 2,5-dihydroxybenzenesulfonic acid, 2-naphthalenesulfonic acid, aspartic acid, benzenesulfonic acid, amphor-10-sulfonic acid, cyclohexylsulfamic acid, dodecylsulfuric acid, ethane-1,2-disulfonic acid, ethanesulfonic acid, fumaric acid, glutamic acid, hydrobromic acid, hydrochloric acid, methanesulfonic acid, naphthalene-1,5-disulfonic acid, nitric acid, phosophoric acid, p-toluenesulfonic acid, sulfuric acid and/or thiocyanic acid; more preferably the salt being a hydrochloride;
or
d) a nonracemic mixtures of (b) and (c).
